## Europäisches Patentamt

## European Patent Office

## Office européen des brevets

(11) Veröffentlichungsnummer: **0 037 501**
**B1**

(12) **EUROPÄISCHE PATENTSCHRIFT**

(45) Veröffentlichungstag der Patentschrift:
**18.07.84**

(21) Anmeldenummer: **81102130.2**

(22) Anmeldetag: **20.03.81**

(51) Int. Cl.³: **A 61 K 31/635,** A 61 K 31/63, A 61 K 9/00, A 61 K 45/06 // (A61K31/635, 31/63, 31/505, 31/115)

(54) Pharmazeutische Präparate und deren Herstellung.

(30) Priorität: **25.03.80 CH 2328/80**

(43) Veröffentlichungstag der Anmeldung:
**14.10.81 Patentblatt 81/41**

(45) Bekanntmachung des Hinweises auf die Patenterteilung:
**18.07.84 Patentblatt 84/29**

(84) Benannte Vertragsstaaten:
**AT BE CH DE FR IT LI LU NL SE**

(56) Entgegenhaltungen:
**CH - A - 247 344**
**CH - A - 544 053**
**DE - C - 836 079**
**DE - C - 954 725**
**US - A - 2 538 557**
**US - A - 2 538 558**
**US - A - 3 985 876**

**CHEMICAL ABSTRACTS, Band 48, Nr. 2, 25. Januar 1954, Columbus, Ohio, U.S.A. R. MEIER et al.: "The curative effect of aldehyde derivatives of sulfonamide compounds"**
**T.W. Greene, "Protective Groups in Organic Synthesis", Wiley-Interscience 1981, S. 293,303,304**

(73) Patentinhaber: **F. HOFFMANN-LA ROCHE & CO. Aktiengesellschaft, CH-4002 Basel (CH)**

(72) Erfinder: **Kompis, Ivan, Dr., Lettenhofstrasse 6, CH-4104 Oberwil (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

BUNDESDRUCKEREI BERLIN

## Beschreibung

Die vorliegende Erfindung betrifft pharmazeutische Präparate auf der Basis von Sulfonamid-Potentiator-Kombinationen.

Kombinationen von Sulfonamiden und Sulfonamid-Potentiatoren (letztere im folgenden der Einfachheit halber Potentiatoren genannt) werden in großem Umfang zur Behandlung von bakteriellen Infektionen in der Human- und Veterenärmedizin eingesetzt. Infolge der unterschiedlichen Löslichkeitscharakteristiken der Sulfonamide und der Potentiatoren und infolge des Umstandes, daß eine schwache Base (Potentiator) mit einer schwachen Säure (Sulfonamid) in nicht stöchiometrischen Mengen kombiniert werden muß, bereitet die Herstellung von pharmazeutisch anwendbaren Lösungen, z. B. Injektionslösungen, von Kombinationen dieser Stoffe Schwierigkeiten. Derartige Injektionslösungen, die größere Mengen organischer Lösungsmittel enthalten, sind Gegenstand der schweizerischen Patentschrift 544 053 und der US-Patentschrift 3 985 876. Die bisher vorgeschlagenen Lösungen konnten nicht vollumfänglich befriedigen, sei es im Hinblick auf die Verträglichkeit der Lösungsmittel, besonders größerer Mengen organischer Lösungsmittel, sei es in bezug auf die Freisetzung der Wirkstoffe oder die Haltbarkeit der Präparate, oder auch im Hinblick auf deren Herstellungskosten. Diesem Mangel soll mit der vorliegenden Erfindung abgeholfen werden, indem pharmazeutische Präparate in wäßriger Form oder in einfacher Weise in wäßrige Form überführbare Präparate bereitgestellt werden, die eine befriedigende Verträglichkeit und Wirksamkeit besitzen, eine hohe Wirkstoffkonzentration und physiologisch günstige pH-Werte aufweisen, ausreichend stabil sind und keine kostspieligen Hilfsstoffe enthalten.

Die Erfindung betrifft somit wäßrige Lösungen auf der Basis eines wasserlöslichen Sulfonamid-Salzes, eines Potentiators und von Formaldehyd, Glykolaldehyd oder Glycerinaldehyd; und Trockenrückstände solcher Lösungen.

Die erfindungsgemäßen Präparate sind nicht einfache Lösungen der drei Komponenten in Wasser. Physikochemische Untersuchungen wie auch die Tatsache, daß sich die Trockenrückstände wieder zu wäßrigen Lösungen rekonstituieren lassen, deuten auf die Bildung einer chemischen Verbindung hin, an der der Aldehyd beteiligt ist. Beispielsweise zeigt ein aus Formaldehyd, Trimethoprim und Sulfadoxin erhaltenes Präparat im $^{13}$C-Kernresonanzspektrum zwei für Methylengruppen charakteristische Signale bei 67,51 und 52,74 ppm und im IR-Spektrum keine für Formaldehyd charakteristische Carbonylbande.

Reaktionsprodukte eines Sulfonamids allein mit einem Aldehyd sind in der schweizerischen Patentschrift 247 344 und den US-Patentschriften 2 538 557 und 2 538 558 beschrieben worden.

Der Ausdruck Potentiator bezeichnet Verbindungen, die die antibakterielle Wirkung von Sulfonamiden mehr als additiv verstärken. Solche Sulfonamid-Potentiatoren sind insbesondere Verbindungen, welche die Dihydrofolat-Reduktase hemmen, wie vorzugsweise 2, 4-Diaminopyrimidinderivate. Beispiele solcher 2,4-Diaminopyrimidinderivate sind im Phenylring substituierte 2,4-Diamino-5-benzylpyrimidine, wie 2,4-Diamino-5-(3,4,5-trimethoxybenzyl)-pyrimidin (Trimethoprim), 2,4-Diamino-5-(3,5-dimethoxy-4-methoxy-äthoxybenzyl)-pyrimidin (Tetroxoprim) und 2,4-Diamino-5-(3,5-dimethoxy-4-methylthiobenzyl)-pyrimidin (Metioprim). Weitere Beispiele von Dihydrofolat-Reduktasehemmern sind 2,4-Diamino-5-(4-brom-3,5-dimethoxybenzyl)-pyrimidin; 2,4-Diamino-5-[3,5-diäthoxy-4-(pyrrol-1-yl)-benzyl]-pyrimidin; 2,4-Diamino-5-(3,4-dimethoxybenzyl)-pyrimidin (Diaveridin); 2,4-Diamino-5-(p-chlorphenyl)-6-äthylpyrimidin (Pyrimethamin) und 2,4-Diamino-5-(2-methyl-4,5-dimethoxybenzyl)-pyrimidin.

Beispiele von Sulfonamiden, die in den erfindungsgemäßen Präparaten enthalten sein können, sind insbesondere $N^1$-heterocyclisch substituierte Sulfonamide, wie solche mit einem 5- oder 6gliedrigen Heterocyclus, wie einem Pyrimidin-, Pyrazin-, Pyridazin-, Oxazol-, Isoxazol-, Thiazol-, Thiadiazol-Ring; spezifische Beispiele von Sulfonamiden sind Sulfadiazin, Sulfamethoxazol, Sulfatroxazol, Sulfamerazin, Sulfadoxin, Sulfadimethoxin, Sulfamethazin, Sulfapyrazol, Sulfachinoxalin, Sulfachlorpyridazin, Sulfaguanidin, Sulfalen, Sulfametin, Sulfamethoxin, Sulfamethoxypyridazin, Sulfamethylphenazol, Sulfaphenazol, Sulfamoxol, Sulfapyrazin, Sulfapyridazin, Sulfapyridin, Sulfasymazin, Sulfathiazol und Sulfametrol.

Von besonderem Interesse als Bestandteil der erfindungsgemäßen Präparate sind die Kombinationen

Sulfamethoxazol/Trimethoprim;
Sulfadiazin/Trimethoprim;
Sulfadoxin/Trimethoprim;
Sulfametrol/Trimethoprim;
Sulfadiazin/Tetroxoprim;
Sulfadoxin/Pyrimethamin.

Der zur Herstellung der erfindungsgemäßen Präparate verwendete Aldehyd ist vorzugsweise Formaldehyd.

Das Molverhältnis von Potentiator : Aldehyd ist zweckmäßig kleiner oder gleich 1 : 1, d. h., pro Mol

2

Potentiator ist ein Mol Aldehyd oder mehr anwesend. Ein bevorzugtes Molverhältnis Potentiator : Aldehyd ist 1 : 1 bis 1 : 4, insbesondere 1 : 1,5 bis 1 : 2,5.

Der Anteil an Sulfonamid in den erfindungsgemäßen Präparaten richtet sich nach der therapeutischen Wirksamkeit der Sulfonamid-Potentiator-Kombinationen. Bei den handelsüblichen Kombinationen ist das Molverhältnis Sulfonamid : Potentiator größer oder gleich 1 : 1, im Falle der handelsüblichen Kombinationen Sulfamethoxazol : Trimethoprim ist es z. B. etwa 5,7 : 1 (entsprechend einem Gewichtsverhältnis von 5 : 1). Ein bevorzugtes erfindungsgemäßes Präparat enthält Sulfamethoxazol, Trimethoprim und Formaldehyd im Molverhältnis von etwa 5,7 : 1 : 2.

Die erfindungsgemäßen Präparate können grundsätzlich durch Vermischen der Bestandteile und Erwärmen, zweckmäßig bis etwa 80°, hergestellt werden, beispielsweise dadurch, daß man zu einer wäßrigen Lösung eines Sulfonamidsalzes einen Potentiator und wäßrige Aldehydlösung gibt und das Gemisch erwärmt, bis vollständige Lösung eingetreten ist. Zweckmäßig löst man das Sulfonamid in Wasser, das die zur Salzbildung erforderliche Menge Base enthält. In einer besonderen Ausführungsform gibt man die wäßrige Aldehydlösung zu der Lösung des Sulfonamidsalzes und gibt dann den Potentiator zu. Als Basen für die Salzbildung kommen insbesondere Alkalihydroxide, wie Natrium- oder Kaliumhydroxid oder pharmazeutisch verwendbare organische Basen, wie N-Methylglucamin, oder basische Aminosäuren, wie Lysin, Arginin oder Ornithin in Betracht.

Die Konzentration der erfindungsgemäßen Lösungen, d. h., der Gehalt an gelösten Stoffen, kann 40 Gew.-% oder mehr betragen, vorzugsweise stellt man 10—20%ige Lösungen her. Bei bestimmten Sulfonamid-Potentiator-Kombinationen kann es bei der Herstellung zweckmäßig sein, der Lösung zusätzlich ein geeignetes, mit Wasser mischbares organisches Lösungsmittel, wie Glycofurol oder Polyäthylenglykol 400, zuzusetzen.

Die erfindungsgemäßen wäßrigen Lösungen können mittels an sich bekannter galenischer Techniken zur Trockene gebracht werden, z. B. durch Gefrier- oder Sprühtrocknung. Die so erhaltenen Trockenpräparate, die ebenfalls Gegenstand der Erfindung sind, können, gegebenenfalls nach vorheriger Sterilisation, durch Zusatz von Wasser wieder in Lösungen, z. B. Injektionslösungen, übergeführt werden.

Die Präparate können weiterhin mittels in der Galenik bei der Herstellung parenteraler Applikationsformen üblichen Techniken sterilisiert werden, z. B. durch Hitzesterilisation oder Sterilfiltration.

Die Verwendungsmöglichkeit der erfindungsgemäßen Präparate beschränkt sich nicht auf Injektionslösungen, die Präparate können auch für andere Zwecke, bei denen eine Sulfonamid-Potentiator-Kombination in gelöster Form eingesetzt werden soll, verwendet werden.

Die erfindungsgemäßen Präparate zeigen in vitro und in vivo die bekannte antibakterielle Aktivität der entsprechenden Sulfonamid-Potentiator-Kombinationen und können daher für die gleichen Indikationen Verwendung finden.

Die nachfolgenden Beispiele erläutern die Erfindung weiter.


## Beispiel 1

800 mg Sulfamethoxazol werden unter Stickstoff bei 40° in 3,15 ml 1N NaOH gelöst. Die Lösung wird mit ca. 5 ml Wasser verdünnt, danach werden 160 mg Trimethoprim und 0,08 ml ca. 35% Formaldehyd-Lösung oder eine äquivalente Menge Paraformaldehyd zugegeben. Die Suspension wird unter Rühren für 5—7 Minuten in ein 80° warmes Bad getaucht, wobei eine klare Lösung entsteht. Nach Abkühlen auf Raumtemperatur wird das Gesamtvolumen auf 10 ml mit Wasser aufgefüllt, wobei eine Lösung von pH 8,8 erhalten wird.


## Beispiel 2

In Analogie zu Beispiel 1 erhält man aus 800 mg Sulfamethoxazol, 3,15 ml 1N NaOH, 160 mg Trimethoprim, 132 mg Glycolaldehyd und Wasser ad 10 ml eine Lösung von pH 8,6.


## Beispiel 3

In Analogie zu Beispiel 1 erhält man aus 1,5 g Trimethoprim, 4,5 g Sulfadiazin, 18,0 ml 1N NaOH, 0,9 ml ca. 35%-Formaldehydlösung und Wasser ad 100 ml eine Lösung von pH 9,1.


## Beispiel 4

In Analogie zu Beispiel 1 erhält man aus 2,0 g Tetroxoprim, 5,0 g Sulfadiazin, 20,0 ml 1N NaOH, 0,85 ml ca. 35%-Formaldehydlösung und Wasser ad 100 ml eine Lösung von pH 9,5.

### Beispiel 5

In Analogie zu Beispiel 1 erhält man aus 4,0 g Trimethoprim, 20,0 g Sulfadoxin, 64,5 ml 1N NaOH, 1,8 ml ca. 35%-Formaldehydlösung und Wasser ad 100 ml eine Lösung von pH 9,1.

### Beispiel 6

In Analogie zu Beispiel 1 erhält man aus 4,0 g Trimethoprim, 20,0 g Sulfatroxazol, 74,8 ml 1N NaOH, 1,5 ml ca. 35%-Formaldehydlösung und Wasser ad 100 ml eine Lösung von pH 9,2.

### Beispiel 7

In Analogie zu Beispiel 1 erhält man aus 4,0 g 2,4-Diamino-5-(4-brom-3,5-dimethoxybenzyl)-pyrimidin, 20,0 g Sulfadimethoxin, 32,0 ml 2N NaOH, 40,0 ml Glycofurol 75, 2,2 ml ca. 35%-Formaldehydlösung und Wasser ad 100 ml eine Lösung von pH 9,6.

### Beispiel 8

In Analogie zu Beispiel 1 erhält man aus 1,6 g Trimethoprim, 8,0 g Sulfametrol, 28,0 ml 1N NaOH, 0,8 ml ca. 35%-Formaldehydlösung und Wasser ad 100 ml eine Lösung von pH 9,6.

### Beispiel 9

In Analogie zu Beispiel 1 erhält man aus 4,08 g Trimethoprim, 20,4 g Sulfamethoxin, 66 ml 1N NaOH und 2,5 ml ca. 35%-Formaldehydlösung und Wasser ad 100 ml eine Lösung von pH 9,6.

### Beispiel 10

In Analogie zu Beispiel 1 erhält man aus 10,86 g einer Molekularverbindung Trimethoprim-Sulfamethoxazol (1 : 1) [F. Giordano, G. P. Bettinetti, A. La Manca, P. Ferloni, Il Pharmaco Ed Sci., 32, 889 (1977)], 20 ml 1N NaOH, 1,3 g Paraformaldehyd (95—97%) und Wasser ad 100 ml eine Lösung von pH 9,75. Diese Lösung wurde bei 25° unter vermindertem Druck (0,1 Torr) zur Trockene eingedampft und der Rückstand 48 Stunden bei Raumtemperatur getrocknet.

Analyse des Rückstandes (2 Analysen)
Gef. C 50,18, 50,19%, H 5,29, 5,16%, N 15,49, 15,69, S 4,98, 5,09
$H_2O$: 3,86%

Dieser Rückstand kann in 100 ml Wasser gelöst werden, um eine Injektionslösung zu rekonstituieren.

### Beispiel 11

In Analogie zu Beispiel 1 erhält man aus 5 g Sulfadoxin 16,1 ml 1N NaOH, 0,25 g Pyrimethamin und 0,80 ml ca. 35%-Formaldehydlösung unter Zugabe von 7,5 ml Polyäthylenglycol 400 und Wasser ad 50 ml eine Lösung von pH 9,1.

### Beispiel 12

320 g Sulfamethoxazol werden unter Stickstoff in ca. 1 l Wasser suspendiert und durch Zugabe von 20% NaOH-Lösung (ad pH 8,8) gelöst. Zu dieser Lösung gibt man 32 ml ca. 35% Formaldehyd-Lösung und das Gemisch wird auf 90° erwärmt, danach werden 64 g Trimethoprim zugegeben und unter Rühren gelöst. Die Lösung wird auf Zimmertemperatur abgekühlt, das pH der Lösung wird auf 9,0 eingestellt und das Gesamtvolumen mit Wasser auf 2 l aufgefüllt. Diese Lösung kann bei —32° lyophilisiert werden (Einfriertemperatur —35°).

## Patentansprüche für die Vertragsstaaten BE, CH, DE, FR, IT, LI, LU, NL, SE

1. Wäßrige Lösungen auf der Basis eines wasserlöslichen Sulfonamid-Salzes, eines Sulfonamid-Potentiators und von Formaldehyd, Glykolaldehyd oder Glycerinaldehyd und Trockenrückstände solcher Lösungen.

2. Lösungen gemäß Anspruch 1, in denen der Aldehyd Formaldehyd ist; und Trockenrückstände solcher Lösungen.

3. Lösungen gemäß den Ansprüchen 1 oder 2, in denen der Potentiator ein Dihydrofolat-Reduktasehemmer ist; und Trockenrückstände solcher Lösungen.

4. Lösungen gemäß Anspruch 3, in denen der Dihydrofolat-Reduktasehemmer ein 2,4-Diamino-pyrimidinderivat ist; und Trockenrückstände solcher Lösungen.

5. Lösungen gemäß Anspruch 4, in denen das 2,4-Diamino-pyrimidinderivat ein 2,4-Diamino-5-benzylpyrimidinderivat ist; und Trockenrückstände solcher Lösungen.

6. Lösungen gemäß Anspruch 5, in denen das 2,4-Diaminopyrimidinderivat Trimethoprim, Tetroxoprim oder Metioprim ist; und Trockenrückstände solcher Lösungen.

7. Lösungen gemäß den Ansprüchen 1—6, in denen das Sulfonamid Sulfamethoxazol, Sulfadiazin, Sulfadioxin oder Sulfametrol ist; und Trockenrückstände solcher Lösungen.

8. Lösungen gemäß den Ansprüchen 1—7, in denen das Molverhältnis Potentiator : Aldehyd kleiner oder gleich 1 : 1 ist; und Trockenrückstände solcher Lösungen.

9. Lösungen gemäß Anspruch 8, in denen das Molverhältnis Potentiator : Aldehyd 1 : 1 bis 1 : 4, besonders 1 : 1,5 bis 1 : 2,5 ist; und Trockenrückstände solcher Lösungen.

10. Lösungen gemäß den Ansprüchen 1—9, in denen das Molverhältnis Sulfonamid : Potentiator größer oder gleich 1 : 1 ist; und Trockenrückstände solcher Lösungen.

11. Lösungen gemäß den Ansprüchen 1—10, auf der Basis eines wasserlöslichen Sulfamethoxazol-Salzes, Trimethoprim und Formaldehyd; und Trockenrückstände solcher Lösungen.

12. Lösungen gemäß Anspruch 11, in denen das Molverhältnis Sulfamethoxazol : Trimethoprim : Formaldehyd etwa 5,7 : 1 : 2 ist; und Trockenrückstände solcher Lösungen.

13. Sterile Injektionslösungen auf der Basis einer Lösung gemäß den Ansprüchen 1—12.

14. Verfahren zur Herstellung von Lösungen gemäß den Ansprüchen 1—13 und von Trockenrückständen solcher Lösungen, dadurch gekennzeichnet, daß man eine wäßrige Lösung eines Sulfonamidsalzes, einen Sulfonamid-Potentiator und wäßrige Aldehydlösung mischt, das Gemisch bis zur vollständigen Lösung erwärmt und gewünschtenfalls die so erhaltene Lösung sterilisiert und/oder zur Trockene bringt und gewünschtenfalls den Trockenrückstand sterilisiert.


## Patentansprüche für den Vertragsstaat AT

1. Verfahren zur Herstellung einer wäßrigen Lösung auf der Basis eines wasserlöslichen Sulfonamid-Salzes, eines Sulfonamid-Potentiators und von Formaldehyd, Glykolaldehyd oder Glycerinaldehyd; und von Trockenrückständen solcher Lösungen, dadurch gekennzeichnet, daß man eine wäßrige Lösung eines Sulfonamidsalzes, einen Sulfonamid-Potentiator und wäßrige Aldehydlösung mischt, das Gemisch bis zur vollständigen Lösung erwärmt und gewünschtenfalls die so erhaltene Lösung sterilisiert und/oder zur Trockene bringt und gewünschtenfalls den Trockenrückstand sterilisiert.

2. Verfahren gemäß Anspruch 1, dadurch gekennzeichnet, daß der Aldehyd Formaldehyd ist.

3. Verfahren gemäß den Ansprüchen 1 oder 2, dadurch gekennzeichnet, daß der Potentiator ein Dihydrofolat-Reduktasehemmer ist.

4. Verfahren gemäß Anspruch 3, dadurch gekennzeichnet, daß der Dihydrofolat-Reduktasehemmer ein 2,4-Diamino-pyrimidinderivat ist.

5. Verfahren gemäß Anspruch 4, dadurch gekennzeichnet, daß das 2,4-Diamino-pyrimidinderivat ein 2,4-Diamino-5-benzylpyrimidin ist.

6. Verfahren gemäß Anspruch 5, dadurch gekennzeichnet, daß das 2,4-Diaminopyrimidinderivat Trimethoprim, Tetroxoprim oder Metioprim ist.

7. Verfahren gemäß den Ansprüchen 1—6, dadurch gekennzeichnet, daß das Sulfonamid Sulfamethoxazol, Sulfadiazin, Sulfadoxin oder Sulfametrol ist.

8. Verfahren gemäß den Ansprüchen 1—6, dadurch gekennzeichnet, daß das Molverhältnis Potentiator : Aldehyd kleiner oder gleich 1 : 1 ist.

9. Verfahren gemäß Anspruch 7, dadurch gekennzeichnet, daß das Molverhältnis Potentiator : Aldehyd 1 : 1 bis 1 : 4, besonders 1 : 1,5 bis 1 : 2,5 ist.

10. Verfahren gemäß den Ansprüchen 1—8, dadurch gekennzeichnet, daß das Molverhältnis Sulfonamid : Potentiator größer oder gleich 1 : 1 ist.

11. Verfahren gemäß den Ansprüchen 1—10, dadurch gekennzeichnet, daß das Sulfonamid Sulfamethoxazol, der Potentiator Trimethoprim und der Aldehyd Formaldehyd ist.

12. Verfahren gemäß Anspruch 11, dadurch gekennzeichnet, daß das Molverhältnis Sulfamethoxazol : Trimethoprim : Formaldehyd etwa 5,7 : 1 : 2 ist.

13. Verfahren zur Herstellung von Injektionslösungen auf der Basis von gemäß Anspruch 1 hergestellten Lösungen, dadurch gekennzeichnet, daß man die Lösung sterilisiert.

**Patent Claims for the Contracting States BE, CH, DE, FR, IT, LI, LU, NL, SE**

1. Aqueous solutions based on a water-soluble sulphonamide salt, a sulphonamide potentiator and formaldehyde, glycol aldehyde or glycerine aldehyde; and dry residues of such solutions.

2. Solutions in accordance with claim 1, in which the aldehyde is formaldehyde; and dry residues of such solutions.

3. Solutions in accordance with claim 1 or 2, in which the potentiator is a dihydrofolate reductase inhibitor; and dry residues of such solutions.

4. Solutions in accordance with claim 3, in which the dihydrofolate reductase inhibitor is a 2,4-diamino-pyrimidine derivative; and dry residues of such solutions.

5. Solutions in accordance with claim 4, in which the 2,4-diamino-pyrimidine derivative is a 2,4-diamino-5-benzylpyrimidine derivative; and dry residues of such solutions.

6. Solutions in accordance with claim 5, in which the 2,4-diaminopyrimidine derivative is trimethoprim, tetroxoprim or metioprim; and dry residues of such solutions.

7. Solutions in accordance with claims 1—6, in which the sulphonamide is sulphamethoxazole, sulphadiazine, sulphadoxine or sulphametrole; and dry residues of such solutions.

8. Solutions in accordance with claims 1—7, in which the molar ratio potentiator : aldehyde is smaller than or equal to 1 : 1; and dry residues of such solutions.

9. Solutions in accordance with claim 8, in which the molar ratio potentiator : aldehyde is 1 : 1 to 1 : 4, especially 1 : 1,5 to 1 : 2,5; and dry residues of such solutions.

10. Solutions in accordance with claims 1—9, in which the molar ratio sulphonamide : potentiator is greater than or equal to 1 : 1; and dry residues of such solutions.

11. Solutions in accordance with claims 1—10 based on a water-soluble sulphamethoxazole salt, trimethoprim and formaldehyde; and dry residues of such solutions.

12. Solutions in accordance with claim 11, in which the molar ratio sulphamethoxazole : trimethoprim : formaldehyde is about 5,7 : 1 : 2; and dry residues of such solutions.

13. Sterile injection solutions based on a solution in accordance with claims 1—12.

14. A process for the manufacture of solutions in accordance with claims 1—13 and of dry residues of such solutions, characterized by mixing an aqueous solution of a sulphonamide salt, a sulphonamide potentiator and aqueous aldehyde solution, warming the mixture until solution is complete and, if desired, sterilizing the thus-obtained solution and/or bringing the solution to dryness and, if desired, sterilizing the dry residue.

**Patent Claims for the Contracting State AT**

1. A process for the manufacture of an aqueous solution based on a water-soluble sulphonamide salt, a sulphonamide potentiator and formaldehyde, glycol aldehyde or glycerine aldehyde; and of dry residues of such solutions, characterized by mixing an aqueous solution of a sulphonamide salt, a sulphonamide potentiator and aqueous aldehyde solution, warming the mixture until solution is complete and, if desired, sterilizing the thus-obtained solution and/or bringing the solution to dryness and, if desired, sterilizing the dry residue.

2. A process in accordance with claim 1, characterized in that the aldehyde is formaldehyde.

3. A process in accordance with claim 1 or 2, characterized in that the potentiator is a dihydrofolate reductase inhibitor.

4. A process in accordance with claim 3, characterized in that the dihydrofolate reductase inhibitor is a 2,4-diamino-pyrimidine derivative.

5. A process in accordance with claim 4, characterized in that the 2,4-diamino-pyrimidine derivative is a 2,4-diamino-5-benzylpyrimidine.

6. A process in accordance with claim 5, characterized in that the 2,4-diaminopyrimidine derivative is trimethoprim, tetroxoprim or metioprim.

7. A process in accordance with claims 1—6, characterized in that the sulphonamide is sulphamethoxazole, sulphadiazine, sulphadoxine or sulphametrole.

8. A process in accordance with claims 1—6, characterized in that the molar ratio potentiator : aldehyde is smaller than or equal to 1 : 1.

9. A process in accordance with claim 7, characterized in that the molar ratio potentiator : aldehyde is 1 : 1 to 1 : 4, especially 1 : 1,5 to 1 : 2,5.

10. A process in accordance with claims 1—8, characterized in that the molar ratio sulphonamide : potentiator is greater than or equal to 1 : 1.

11. A process in accordance with claims 1—10, characterized in that the sulphonamide is sulphamethoxazole, the potentiator is trimthoprim and the aldehyde is formaldehyde.

12. A process in accordance with claim 11, characterized in that the molar ratio sulphamethoxazole : trimethoprim : formaldehyde is about 5,7 : 1 : 2.

13. A process for the manufacture of injection solutions based on solutions manufactured in accordance with claim 1, characterized by sterilizing the solution.

**Revendications pour les Etats contractants BE, CH, DE, FR, IT, LI, LU, NL, SE**

1. Solutions aqueuses à base d'un sel hydrosoluble de sulfonamide, d'un agent potentialisant des sulfonamides et de formaldéhyde, d'aldéhyde glycolique ou d'aldéhyde glycérique, et résidus secs de telles solutions.

2. Solutions selon la revendication 1, dans lequelles l'aldéhyde est le formaldéhyde; et résidus secs de telles solutions.

3. Solutions selon les revendications 1 ou 2, dans lesquelles l'agent potentialisant des sulfonamides est un inhibiteur de la dihydrofolate-réductase; et résidus secs de telles solutions.

4. Solutions selon la revendication 3, dans lesquelles l'inhibiteur de la dihydrofolate-réductase est un dérivé de 2,4-diamino-pyrimidine; et résidus secs de telles solutions.

5. Solutions selon la revendication 4, dans lesquelles le dérivé de 2,4-diamino-pyrimidine est un dérivé de 2, 4-diamino-5-benzylpyrimidine; et résidus secs de telles solutions.

6. Solutions selon la revendication 5, dans lesquelles le dérivé de 2,4-diamino-pyrimidine est le Trimethoprim, le Tetroxoprim ou le Metioprim; et résidus secs de telles solutions.

7. Solutions selon les revendications 1 à 6, dans lesquelles le sulfonamide est le Sulfamethoxazol, le Sulfadiazin, le Sulfadoxin ou le Sulfametrol; et résidus secs de telles solutions.

8. Solutions selon les revendications 1 à 7, dans lesquelles le rapport molaire agent potentialisant/aldéhyde est inférieur ou égal à 1 : 1; et résidus secs de telles solutions.

9. Solutions selon la revendication 8, dans lesquelles le rapport molaire agent potentialisant/aldéhyde va de 1 : 1 à 1 : 4, plus particulièrement de 1 : 1,5 à 1 : 2,5; et résidus secs de telles solutions.

10. Solutions selon les revendications 1 à 9, dans lesquelles le rapport molaire sulfonamide/agent potentialisant est supérieur ou égal à 1 : 1; et résidus secs de telles solutions.

11. Solutions selon les revendications 1 à 10, à base d'un sel hydrosoluble de Sulfamethoxazol, de Trimethoprim et de formaldéhyde; et résidus secs de telles solutions.

12. Solutions selon la revendication 11, dans lesquelles les proportions molaires Sulfamethoxazol/Trimethoprim/formaldéhyde sont d'environ 5,7 : 1 : 2; et résidus secs de telles solutions.

13. Solutions stériles pour injections à base d'une solution selon les revendications 1 à 12.

14. Procédé de préparation de solutions selon les revendications 1 à 13 et de résidus secs de telles solutions, caractérisé en ce que l'on mélange une solution aqueuse d'un sel de sulfonamide, un agent potentialisant des sulfonamides et une solution aqueuse d'aldéhyde, on chauffe le mélange jusqu'à dissolution complète et si on le désire, on stérilise la solution obtenue et/ou on l'amène à sec, et si on le désire, on stérilise le résidu sec.

**Revendications pour l'Etat contractant AT**

1. Procédé de préparation d'une solution aqueuse à base d'un sel hydrosoluble de sulfonamide, d'un agent potentialisant des sulfonamides et de formaldéhyde, d'aldéhyde glycolique ou d'aldéhyde glycérique; et de résidus secs de telles solutions, caractérisé en ce qu'on mélange une solution aqueuse d'un sel de sulfonamide, un agent potentialisant des sulfonamides et une solution aqueuse d'aldéhyde, on chauffe le mélange jusqu'à dissolution complte et si on le désire, on stérilise la solution obtenue et/ou on l'amène à sec, et si on le désire, on stérilise le résidu sec.

2. Procédé selon la revendication 1, caractérisé en ce que l'aldéhyde est le formaldéhyde.

3. Procédé selon les revendications 1 ou 2, caractérisé en ce que l'agent potentialisant est un inhibiteur de la dihydrofolate-réductase.

4. Procédé selon la revendication 3, caractérisé en ce que l'inhibiteur de la dihydrofolate-réductase est un dérivé de 2,4-diamino-pyrimidine.

5. Procédé selon la revendication 4, caractérisé en ce que le dérivé de 2,4-diamino-pyrimidine est une 2,4-diamino-5-benzyl-pyrimidine.

6. Procédé selon la revendication 5, caractérisé en ce que le dérivé de 2,4-diamino-pyrimidine est le Trimethoprim, le Tetroxoprim ou le Metioprim.

7. Procédé selon les revendications 1 à 6, caractérisé en ce que le sulfonamide est le Sulfamethoxazol, le Sulfadiazin, le Sulfadoxin ou le Sulfametrol.

8. Procédé selon les revendications 1 à 6, caractérisé en ce que le rapport molaire agent potentialisant/aldéhyde est inférieur ou égal à 1 : 1.

9. Procédé selon la revendication 7, caractérisé en ce que le rapport molaire agent potentialisant/aldéhyde va de 1 : 1 à 1 : 4, plus spécialement de 1 : 1,5 à 1 : 2,5.

10. Procédé selon les revendications 1 à 8, caractérisé en ce que le rapport molaire sulfonamide/agent potentialisant est supérieur ou égal à 1 : 1.

11. Procédé selon les revendications 1 à 10, caractérisé en ce que le sulfonamide est le Sulfamethoxazol, l'agent potentialisant est le Trimethoprim et l'aldéhyde est le formaldéhyde.

12. Procédé selon la revendication 11, caractérisé en ce que les proportions molaires Sulfamethoxazol/Trimethoprim/formaldéhyde sont d'environ 5,7 : 1 : 2.

13. Procédé de préparation de solutions pour injections à base des solutions préparées selon la revendication 1, caractérisé en ce que l'on stérilise la solution.